# EUROPEAN PATENT APPLICATION

(11) **EP 4 480 481 A1**
(43) Date of publication of application: **25.12.2024**
(21) Application number: 23305992.2
(22) Date of filing: 22.06.2023
(51) Int. Cl.: A61K 31/4402, A61P 1/02, A61K 9/00

(54) **MEPYRAMINE FOR USE IN THE TOPICAL TREATMENT OF ORAL MUCOSITIS**

(71) Applicant: ASSISTANCE PUBLIQUE-HOPITAUX DE PARIS, 75012 Paris 12 (FR); Centre National de la Recherche Scientifique, 75016 Paris (FR)
(72) Inventor: GRECO, Céline, 75012 PARIS (FR); DELMAS, Patrick, 13880 VELAUX (FR)
(74) Representative: Gevers & Orès

(57) **Abstract**

The present invention relates to a compound selected from mepyramine, prodrugs thereof and pharmaceutically acceptable salts thereof, or a composition comprising such a compound, for use in the topical treatment and prevention of oral mucositis.

## Description

The present invention relates to a compound selected from mepyramine, prodrugs thereof and pharmaceutically acceptable salts thereof, or a composition comprising such a compound, for use in the topical treatment and prevention of oral mucositis

Oral mucositis is a severely incapacitating condition characterized by erythema, edema, and ulcerations of the oral mucosa. It is probably the most common adverse effect of radiation therapy, chemotherapy, chemoradiotherapy and hematopoietic stem cell transplantation. It can cause several problems, including pain, nutritional problems as a result of inability to eat, and increased risk of infection due to open sores in the mucosa. It has a significant effect on the patient's quality of life and can be dose-limiting for anticancer treatments (i.e. requiring a reduction in subsequent chemotherapy doses). The lesions can also break the mucosa barrier resulting in local or systemic infection. In severe cases, this can lead to parenteral nutrition, ultimately leading to poor quality of life.

About 30-40% of the patients treated with chemotherapy develop mucositis; this percentage rises to 60-85% for patients undergoing to an hematopoietic stem cell transplantation and to almost 90% for head and neck cancer patients treated with both chemo- and radiotherapy. 19% of the latter will be hospitalized and will experience a delay in anticancer treatment for high-grade mucositis management, resulting in a reduction of the quality of life, a worse prognosis and an increase in patient management costs. Currently, several interventions and prevention guidelines are available, but their effectiveness is uncertain.

There are no effective treatments for mucositis. Current treatments are generally palliative and include maintaining a high level of oral hygiene, the use of analgesics, such as lidocaine, and mouthwashes, such as chlorohexidine gluconate. Further therapies include the use of agents which reduce the mucosal absorption of chemotherapy drugs, for example cryotherapy or allopurinol. Other treatments include laser therapy and antibiotics, as well as the use of cytokine-based therapies. None of the currently used therapeutic approaches has proved entirely effective in the prophylaxis or treatment of mucositis.

There thus remains a genuine need for effective approaches to preventing and treating oral mucositis for improving the care of cancer patients. Such new approaches will be particularly beneficial to patients with cancerous conditions who will undergo, or who are undergoing, a cancer therapy such as chemotherapy and/or radiotherapy.

The present invention is believed to meet such need by providing a topical composition for treating or preventing oral mucositis.

Mepyramine is a first generation antihistamine of formula (I):

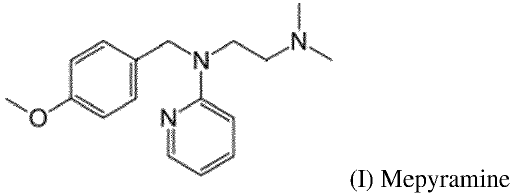

Mepyramine is classically used as a histamine H1 receptor inverse agonist. It binds to a G protein-coupled form of the receptor and promotes a G protein-coupled inactive state of the H1 receptor that interferes with the Gq/11-mediated signaling. Mepyramine competes with histamine for binding at H1-receptor sites on the effector cell surface, resulting in suppression of histaminic edema, flare, and pruritus.

The Inventors have in a surprising manner demonstrated that mepyramine by its inhibitory action on Nav1.7, Nav1.8 and Nav1.9, sodium channels of nociceptors, leads to effective pain relief when applied locally at the level of the oral mucosa, with a low systemic passage (i.e. limiting or avoiding side effects) and a duration of action sufficient for food resumption.

Thus, the present invention concerns a compound selected from mepyramine, prodrugs thereof and pharmaceutically acceptable salts thereof, for use in the topical treatment or prevention of oral mucositis.

The present invention further relates to a topical composition comprising a compound according to the invention as active ingredient and at least one pharmaceutically acceptable excipient, for use in the treatment or prevention of oral mucositis.

The present invention further relates to a method for treating and/or preventing oral mucositis by means of topical administration, to a patient in need thereof, of an effective amount of a compound or composition according to the invention.

The present invention further relates to the use of a compound or composition comprising a compound according to the invention for the manufacture of a topical medication for treating and/or preventing oral mucositis.

"Mepyramine" (also called "pyrilamine") as used herein refers to the molecule N,N-dimethyl-N'-(4-methoxybenzyl)-N'-(2-pyridyl) ethylenediamine, enantiomers, racemic mixtures, polymorphs, salts, solvates, esters or hydrates thereof.

In the present invention, "prodrug" designates a compound that, after administration, is metabolized (*i.e.* converted within the body) into a pharmacologically active drug. In the present invention, the expression "a prodrug of mepyramine" designates any compound that, after administration, is metabolized into a mepyramine.

In the present invention, "pharmaceutically acceptable" is intended to mean that which is useful in the preparation of a pharmaceutical composition, generally safe, nontoxic and neither biologically nor otherwise undesirable, and acceptable for both veterinary and human pharmaceutical use.

"Pharmaceutically acceptable salt" of a compound is intended to mean a salt that is pharmaceutically acceptable, as defined herein, and that has the desired pharmacological activity of the parent compound.

Salts of mepyramine include salts of acidic or basic groups present in compounds of the application. Pharmaceutically acceptable salts of mepyramine include, but are not limited to, mepyramine maleate (which is commercially available under the Trade Name "Anthisan") or pyrilamine acetate.

In an embodiment, the oral mucositis is caused by a radiation therapy, a chemotherapy, a chemoradiotherapy or a hematopoietic stem cell transplantation.

In an embodiment, the oral mucositis is caused by a radiation therapy, in particular a radiation therapy to the head and neck.

In an embodiment, the oral mucositis is caused by a chemotherapy.

In an embodiment, the oral mucositis is caused by a chemoradiotherapy.

In an embodiment, the oral mucositis is caused by a hematopoietic stem cell transplantation.

By "treatment" is meant, according to the present invention, the inhibition of the development of, more particularly the regression of, preferably the disappearance of the pain caused by mucositis.

By "prevention" is meant, according to the present invention, to prevent or delay the appearance of the pain caused by mucositis.

The treatment or prevention according to the invention applies to humans or animals.

By "topical" is meant, according to the present invention, that the compound or composition according to the invention will be administered by application on the mucous membranes of the mouth or the throat or on the skin.

The topical composition according to the invention may in particular be in any form allowing topical application, such as a spray (e.g. an aerosol spray), a cream, a gel, a lozenge, a pastille, an ointment, a solution, a lotion, an aerosol foam or a patch.

In a preferred embodiment, the composition according to the invention will be in the form of a spray.

In an embodiment, the composition of the invention can also be dispensed from an aerosol container.

In an embodiment, the topical composition of the invention comprises a compound according to the invention in a concentration from 1% to 50%, in particular from 5% to 40%, more particularly 10% to 20% by weight relative to the weight of the final composition.

In an embodiment, the topical composition of the invention comprises a compound according to the invention in a concentration of 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% or 50% by weight relative to the weight of the final composition.

In an embodiment, the topical composition of the invention is in the form of a spray and comprises a compound according to the invention in a concentration from 1% to 50%, preferably from 5% to 40%, more preferably from 10% to 20% by weight relative to the weight of the final composition.

In an embodiment, the topical composition of the invention is in the form of a spray and comprises a compound according to the invention in a concentration of 2% by weight relative to the weight of the final composition..

In an embodiment, the topical composition of the invention is in the form of a spray and comprises a compound according to the invention in a concentration of 5% by weight relative to the weight of the final composition.

In an embodiment, the topical composition of the invention is in the form of a spray and comprises a compound according to the invention in a concentration of 10% by weight relative to the weight of the final composition.

In an embodiment, the topical composition of the invention is in the form of a spray and comprises a compound according to the invention in a concentration of 20% by weight relative to the weight of the final composition.

In an embodiment, the topical composition of the invention is in the form of a spray and comprises a compound according to the invention in a concentration of 30% by weight relative to the weight of the final composition.

In an embodiment, the topical composition of the invention is in the form of a spray and comprises a compound according to the invention in a concentration of 40% by weight relative to the weight of the final composition.

In an embodiment, the topical composition of the invention is in the form of a spray and comprises a compound according to the invention in a concentration of 50% by weight relative to the weight of the final composition.

In an embodiment, the topical composition of the invention is formulated into unit dose form.

The compound or topical composition according to the invention will be administered one or more times per day, the duration being variable according to the pain intensity and easily adjustable by the person skilled in the art or the practitioner.

In an embodiment, the topical composition of the invention is applied to the affected area once daily, twice daily, three times daily, once every second day, three times weekly, twice weekly or once weekly.

In an embodiment, the topical composition of the invention is applied before a pain episode.

In an embodiment, the topical composition of the invention is applied during a pain episode.

In an embodiment, the topical composition of the invention is applied before a meal.

In an embodiment, the compound according to the invention topical composition is administered at a dose from 10 to 50 mg, preferably from 20 to 40 mg per application.

In an embodiment, the compound according to the invention topical composition is administered at a dose from 10, 15, 20, 25, 30, 40, 45 or 50 mg per application.

By "pharmaceutically acceptable excipient" is meant, according to the invention, an excipient that is compatible with the other ingredients of the composition and that produces no adverse effect, allergic reaction or other undesirable reaction when it is administered to a human or an animal.

In an embodiment, the topical composition of the invention comprises a gellant, such as carbopol, triethanolamine, preferably a thermo-reversible gellant, such as Poloxamer 407.

In an embodiment, the topical composition of the invention comprises a muco-adhesive agent, such as hypromellose, Novafilm^{®} gel base, Mucolox^{®} or carbomer.

In an embodiment, the topical composition of the invention comprises a flavoring agent, such as orange aroma or cherry aroma.

In an embodiment, the topical composition of the invention comprises an agent that reduces bitterness.

In an embodiment, the topical composition of the invention comprises a sweetener, such as saccharin sodium, aspartame or sucralose.

In an embodiment, the topical composition of the invention comprises a preservative, such as methylparaben or sodium benzoate.

In an embodiment, the topical composition of the invention comprises a diluent such as water or serum bicarbonate, in particular a lipophilic diluent such as fluid paraffin.

In an embodiment, the topical composition of the invention comprises an anti-burn agent such as curcumin or quercetin.

In particular embodiment, the topical composition of the invention comprises quercetin and further comprises Transcutol^{®} as a solvent for quercetin.

In an embodiment, the topical composition of the invention comprises an anti-irritant agent such as sodium hyaluronate.

In an embodiment, the topical composition of the invention comprises a surfactant such as Labrafil^{®} 1944.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### EXAMPLES

Ten patients with a myelogenous leukemia, an acute lymphocytic leukemia, a myeloma or an immune deficiency and suffering from a mucositis were treated.

All patients stopped eating due to pain.

Different forms and doses of mepyramine were tested as follows:
- a muco-adhesive gel of mepyramine 2% was tested (20 mg per drop of 1g; one drop by application).
- a muco-adhesive solution of mepyramine 5% was tested (5 mg by pulverization of 0.1 ml; four pulverizations per application).
- a first thermo-reversible gel of mepyramine 5% was tested (5 mg by pulverization of 0.1 ml; four pulverizations par application).
- a second thermo-reversible gel of mepyramine 5% was tested (5 mg by pulverization of 0.1 ml; four pulverizations par application).
- a third thermo-reversible gel of mepyramine 10% was tested (10 mg by pulverization of 0.1 ml; four pulverizations par application).

The patients were treated for six days on average (three applications of mepyramine per day).

Mean intensity of pain before lunch application was determined by Numeric Rate Scale (NRS) as 6/10.

Mean intensity of pain after lunch application was determined by NRS as 1.9/10.

The duration of efficacy was 120 minutes on average.

The tested treatments allowed a direct resumption of eating for 50% of the patients.

## Claims

1. A compound selected from mepyramine, prodrugs thereof and pharmaceutically acceptable salts thereof, for use in the topical treatment or prevention of oral mucositis.

2. A topical composition comprising a compound selected from mepyramine, prodrugs thereof and pharmaceutically acceptable salts thereof, for use in the treatment or prevention of oral mucositis.

3. The topical composition for use according to claim 2, wherein the oral mucositis is caused by a radiation therapy, a chemotherapy, a chemoradiotherapy or a hematopoietic stem cell transplantation.

4. The topical composition for use according to any one of claims 2-3, wherein the composition is in the form of a spray (e.g. an aerosol spray), a cream, a gel, a lozenge, a pastille, an ointment, a solution, a lotion, an aerosol foam or a patch, preferably a spray.

5. The topical composition for use according to any one of claims 2-4, wherein the compound is in a concentration from 1% to 50%, preferably in a concentration from 5% to 40%, more preferably in a concentration of from 10% to 20%, by weight relative to the weight of the final composition.

6. The topical composition for use according to any one of claims 2-5, wherein the topical composition is applied to the affected area once daily, twice daily, three times daily, once every second day, three times weekly, twice weekly or once weekly.

7. The topical composition for use according to any one of claims 2-6, further comprising a penetration enhancer.
